# EUROPEAN PATENT SPECIFICATION

(11) **EP 2 996 665 B1**
(45) Date of publication and mention of the grant of the patent: **07.09.2016**
(21) Application number: 14722219.4
(22) Date of filing: 08.05.2014
(51) Int. Cl.: A61Q 11/00, A61K 8/19, A61K 8/27, A61K 8/365, A61K 8/73

(54) **ORAL CARE COMPOSITIONS**
MUNDPFLEGEMITTEL
COMPOSITIONS DE PROTECTION ORALE

(30) Priority: 15.05.2013 EP 13167917; 31.05.2013 EP 13169967
(43) Date of publication of application: 23.03.2016
(73) Proprietor: Unilever PLC, a company registered in England and Wales under company no. 41424, London EC4Y 0DY (GB); Unilever N.V., 3013 AL Rotterdam (NL)
(72) Inventor: ANTONELLI CAMILLO, Natalie, 13271450 São Paulo (BR); COLLINS, Luisa, Zoe, Wirral Merseyside CH63 3JW (GB); GUADALUPE GUIMARÃES da SILVA, Rafaela, 13271450 São Paulo (BR); MOUTINHO MONTEIRO, Karen, Cristina, NL-3133 AT Vlaardingen (NL)
(74) Representative: Tansley, Sally Elizabeth
(86) International application number: PCT/EP2014/059432
(87) International publication number: WO 2014/184083

(56) References cited:
- WO-A1-2012/087324
- KR-A- 20110 053 021
- US-A- 4 289 754
- US-A- 4 376 115
- US-A- 5 587 147
- US-A1- 2006 140 876
- US-A1- 2007 183 989
- US-A1- 2012 020 897

## Description

### Field of the Invention

The present invention is concerned with mouthwash oral care compositions. present invention is concerned with mouthwash compositions

### Background of the invention

One of the functions of a mouthwash and similar products is to remove unpleasant mouth odours and of making the user feel confident that their breath will not offend. However there remains a need to further mitigate mouth odours, especially from mouthwashes that are marketed as having mild formulations. Mild formulations contain low levels/no alcohol and no or low levels of anionic surfactants.

Mouthwashes containing gelling agents are disclosed in KR20110053021 and WO02067883.

The present invention relates to a mouthwash that mitigates unpleasant mouth odours.

### Summary of the invention

The present invention is an aqueous based mouthwash oral care composition comprising:
i) a metal salt selected from citrate, lactate or chloride,
ii) a zinc salt,
iii) gellan gum, and
iv) xanthan gum,
in which the composition at a temperature of 25°C has a yield stress from 0.05 to 1.0 Pa and a viscosity from 0.05 to 0.25 Pa.s measured at a shear rate of 21 1/s.

In the context of the present invention the yield stress is defined as the shear stress at a shear rate of 0.1 1/s at 25°C.

The invention further relates to a method of reducing mouth odour by rinsing the mouth and teeth with a composition described above.

### Detailed Description of the invention

The compositions of the invention are in the form of a mouthwash. The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated.

A mouthwash composition according to the invention will usually contain an aqueous continuous phase. The amount of water generally ranges from 70 to 99% by weight based on the total weight of the mouthwash.

A mouthwash composition according to the invention will generally contain further ingredients to enhance performance and/or consumer acceptability, such as the humectants and surfactants. The amount of humectant generally ranges from 4 to 20%, more preferably from 7 to 15% by weight based on the total weight of the mouthwash. Preferred humectants that are present at the above levels include polyols, particularly preferred is sorbitol.

Pouring viscosities and yield stress measurements are made using a thermal Analysis Discovery Hybrid Rheometer 2 (TA DHR2). The measuring geometry used on the rheometer was a 4cm diameter cone with a 2° angle. All measurements were made at 25°C.

The rheometer was used to determine the viscosity at a shear rate of 21 1/s which is taken to be the pouring shear rate. This is based on delivering a 20ml dose in 2s from a 22mm diameter bottle where the flooded depth at the exit of the neck is 10mm. Here the wall shear rate at the exit of the bottle will be of the order of 21 1/s.

In order to determine the yield stress the sample was measured using a stepped stress ramp between stresses of 0.01 Pa and 10Pa with 20 points per decade in stress. The shear rate was determined at each point over a 10s interval. The yield stress is taken as being the stress at which the shear rate is 0.1 1/s.

The composition at a temperature of 25°C has a yield stress from 0.05 to 1.0 Pa and a viscosity from 0.05 to 0.25 Pa.s measured at a shear rate of 21 1/s.

Preferably, at a temperature of 25°C it has a yield stress from 0.1 to 0.5 Pa, and a viscosity from 0.07 to 0.15 Pa.s measured at a shear rate of 21 1/s.

The composition comprises a mixture of gellan gum and xanthan gum. Preferably the weight ratio of xanthan gum to gellan gum is 2:1 to 6: 1; more preferably from 3:2 to 5: 1.

It is preferred if the level of xanthan gum is from 0.02 to 0.5 wt% of the total composition, more preferably from 0.05 to 0.4 wt%.

It is preferred if the level of gellan gum is from 0.02 to 0.4 wt% of the total composition, more preferably from 0.03 to 0.2 wt%.

Compositions of the invention comprise a metal salt selected from citrate, lactate or chloride, preferably the metal salt is a citrate salt, more preferred citrate salts are sodium and potassium citrate; particularly preferred is potassium citrate. It is preferable if the levels of citrate salt are from is from 0.1 to 1 wt% of the total formulation, more preferably from 0.2 to 0.6 wt% of the total composition.

Compositions of the invention comprise a zinc salt, preferably zinc sulphate or zinc chloride, more preferably zinc sulphate heptahydrate. Preferably the level of zinc salt is from 0.05 to 1.0 wt% of the total formulation more preferably from 0.1 to 0.5 wt%.

It is preferable if the level of ethanol in the total composition is less than 0.1 wt%.

Compositions of the invention may comprise a preservative. A preferred preservative is sodium benzoate. Preferably the level of preservative is from 0.1 to 1 wt% of the total composition.

It is preferable if the pH of the composition at 20° C is from 4 .2 to 5.9, more preferably 4.4 to 5.3.

The composition of the invention may comprise a deposition aid. The term "deposition aid" in the context of this invention generally means a material which aids deposition of anti-bacterial agents from the composition.

Use of the composition in the context of this invention typically involves application of the composition to the oral cavity, for a recommended time period before being expectorated. Preferred application times are from 10 to 50 seconds.

Suitable deposition aids for use in the invention will generally dissolve or disperse in water at a temperature of 25°C.

Preferred deposition aids for use in the invention are water soluble. The term "water-soluble" in this particular context generally means that the deposition aid has an aqueous solubility of at least 10g/L at 25°C, and more preferably at least 30g/L at 25°C (where the solubility is determined in un-buffered distilled water). It is particularly preferable that the deposition aid remains water soluble after drying, so that it can be re-dissolved. This prevents undesirable build up of the deposition aid on the teeth after repeated usage of the composition.

Suitable deposition aids for use in the invention include polymeric materials, preferably polymeric materials which are water soluble as defined above.

Polymeric materials for use as deposition aids in the invention may be naturally or synthetically-derived, and may be ionic or nonionic in nature.

Preferably such polymeric materials are high molecular weight. The term "high molecular weight" in this particular context generally means that the polymeric material has a molecular weight of at least 50,000, more preferably at least 500,000 g/mol. A suitable method to determine the molecular weight of such polymeric materials is gel permeation chromatography against a polyethylene glycol standard.

Specific examples of suitable classes of polymeric material for use as deposition aids in the invention include:
Water-soluble, high molecular weight linear homopolymers of ethylene oxide characterised by the general formula H(OCH₂CH₂)ₙOH. These materials are generally termed polyethyleneoxides (or alternatively, polyoxyethylenes, or polyethylene glycols). In the general formula, n usually has an average value of at least 2000, preferably at least 50,000.
Water-soluble, high molecular weight cellulose ethers such as methylcellulose, hydroxyethylcellulose, hydroxypropylcellulose, hydroxypropyl methylcellulose, hydroxybutyl methylcellulose, hydroxyethyl ethylcellulose, sodium carboxymethylcellulose, and sodium carboxymethyl hydroxyethylcellulose.

A preferred class of polymeric material for use as deposition aids in the invention includes water-soluble, high molecular weight polymers having anionic side groups along the polymer main chain.

Specific examples of such materials include poly(carboxylic acid) polymers. Poly (carboxylic acid) polymers are typically polymers which include -COOH groups in their structure, or groups which are derived from -COOH groups such as salt, ester or anhydride groups.

For example, the poly(carboxylic acid) polymers may include:

-[C(R¹)(COOH)-]-

units in their structure, in which R¹ is selected from hydrogen, C₁₋₃ alkyl, C₁₋₃ alkoxy or C₁₋₃ hydroxyalkyl. Preferably R¹ is hydrogen.

A preferred type of poly (carboxylic acid) polymer includes adjacent:

-[C(R¹)(COOH)-]-

units in its structure (where R¹ is as defined above), for example polymers based on maleic acid, which typically include:

-{-CH(COOH)-CH(COOH)-]-

units, and/or salts or esters of such units, or such units in anhydride form in which - COOH groups on adjacent carbon atoms are cyclised to form a ring system.

For example, the poly(carboxylic acid) polymers may comprise units with pairs of carboxylic acid groups on adjacent polymer chain carbon atoms, for example polymers comprising:

-[-C(R¹)(R²)-C(R³)(R⁴)-C(R⁵)(COOH)-C(R⁶)(COOH)-]-

units in its structure (and/or salts or esters of such units, or such units in anhydride form in which -COOH groups on adjacent carbon atoms are cyclised to form a ring system); in which R¹,R²,R³,R⁴,R⁵ and R⁶ are each independently selected from hydrogen, C₁₋₃ alkyl or C₁₋₃ alkoxy. Preferably R¹ and R² are hydrogen, R³ is hydrogen and R⁴ is methoxy and R⁵ and R⁶ are hydrogen.

Such a poly(carboxylic acid) polymer may be described as the polymer based on a copolymer of methyl vinyl ether and maleic anhydride, and is commercially available for example under the trade name Gantrez®.

A particularly preferred example of such a polymer comprises:

-[-CH₂-CH(OCH₃)-CH(COOH)-CH(COOH)-]-

units in its structure, in which the -COOH groups are in free acid form. Such polymers may be linear or cross-linked. More preferably the polymer is linear. Polymers of this type are commercially available for example under the trade name Gantrez® S. Most preferably such a polymer has a molecular weight of at least 500,000 g/mol (e.g. Gantrez® S-96), ideally at least 1,000,000 g/mol (e.g. Gantrez® S-97).

Alternative polymers which may be used comprise the units as described above in anhydride form, i.e. in which the two adjacent -COOH groups are cyclised to form a ring system. Such polymers are commercially available under the tradename Gantrez® AN, e.g. Gantrez® AN-119, Gantrez® AN-903, Gantrez® AN-139 and Gantrez® AN-169.

Other alternative polymers which may be used comprise the units as described above in partial salt form, for example in which some of the free -COOH groups are converted into a metal salt of a Group I or Group II metal such as either sodium or calcium, or a mixed sodium-calcium salt. Such polymers are commercially available under the tradename Gantrez® MS, e.g. Gantrez® MS-955.

Other alternative polymers which may be used comprise the units as described above in partial ester form, for example in which some of the free -COOH groups are esterified with C₁₋₆ alkyl, e.g. ethyl or n-butyl. Such polymers are commercially available under the tradename Gantrez® ES, e.g. Gantrez® ES-225 or Gantrez® ES-425.

Mixtures of any of the above described materials may also be used.

The amount of deposition aid (as defined above) in compositions of the invention suitably ranges from 0.001 to 5.0%, preferably from 0.005 to 4.0%, more preferably from 0.01 to 2.0% by total weight deposition aid (as defined above) based on the total weight of the composition.

Although not preferred the composition may contain low levels of surfactant based on the total weight of the composition. If present, the surfactant is preferably present at levels of less than 0.5 wt% of the total composition, more preferably at levels less than 0.35 wt%, most preferably at levels of less than 0.1 wt%.

Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides.

Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Compositions of the present invention may also contain further optional ingredients customary in the art such as fluoride ion sources, anticalculus agents, buffers, flavouring agents, sweetening agents, colouring agents, opacifying agents, antisensitivity agents and antimicrobial agents.

The invention is further illustrated with reference to the following, non-limiting Examples. Examples according to the invention are illustrated by a number, comparative Examples are illustrated by a letter.

### EXAMPLE

### Formulations

Mouthwash compositions having the ingredients shown in Table 1 below were prepared.

All ingredients are expressed by weight percent of the total formulation, and as level of active ingredient.

**Table 1**

| **Ingredients** | **Wt %** | | | |
|---|---|---|---|---|
| | **Example 1** | **Example 2** | **Example A** | **Example B** |
| Sorbitol | 12.00 | 12.00 | 12.00 | 12.00 |
| Sodium Fluoride | 0.05 | 0.05 | 0.05 | 0.05 |
| PEG-40 Hydrogenated castor oil | 2.5 | 2.5 | 2.5 | 2.5 |
| Aroma | 0.4 | 0.4 | 0.4 | 0.4 |
| Sodium saccharin dihydrated | 0.06 | 0.06 | 0.06 | 0.06 |
| Zinc sulphate heptahydrate | 0.2 | 0 | 0.2 | 0 |
| Glycine | 0.5 | 0.5 | 0.5 | 0.5 |
| Potassium citrate | 0.55 | 0.55 | 0.55 | 0.55 |
| Sodium benzoate | 0.5 | 0.5 | 0.5 | 0.5 |
| Xanthan gum | 0.20 | 0.20 | 0 | 0 |
| Gellan gum | 0.05 | 0.05 | 0 | 0 |
| Water and minors | To 100 | | | |

Example 2 not in accordance with the invention.

Mouthwash compositions were tested for ability to limit production of salivary thiols. Pooled saliva sediment was exposed to undiluted mouthwash for 30 seconds, washed and incubated with fresh pooled saliva overnight (for 24 hours) at 37°C. After incubation, concentration of salivary thiols was calculated using a colourmetric assay. 4, 4'-bis (dimethyl amino) diphenyl carbinol [BDC-OH] was used to quantify the level of salivary thiols generated by each saliva sample.

Colour Reaction:

BDC⁺ + R-SH → BDC-S-R + H⁺

Blue → Colourless

The higher the concentration of BDC-OH remaining, the lower the concentration of salivary thiols in the incubated saliva samples.

**Table 2**

| **Test Agent** | **Mean (µM BDC⁺)** | **Significance (Tukey-Kramer)*** |
|---|---|---|
| Example 1 | 11.1908 | A |
| Example 2 | 8.8923 | B |
| Example A | 8.0741 | C |
| Example B | 6.7136 | D |

| | | |
|---|---|---|
| * Different letter denotes statistical significance, where p<0.05 | | |

Results were significantly different (p<0.05).

The results show that compositions according to the invention have higher concentration of BDC⁺ remaining after treatment and hence lower concentration of salivary thiols, a key component of oral malodour.

Yield stress and pouring viscosities for mouthwash compositions shown in Table 1 were measured using cone and plate rheometer. Results included in Table 3.

**Table 3**

| **Test Agent** | **Yield stress (Pa)** | **Pouring Viscosity* (Pa.s)** |
|---|---|---|
| Example 1 | 0.213 | 0.097 |
| Example 2 | 0.138 | 0.099 |
| Example A | Not applicable | 0.003 |
| Example B | Not applicable | 0.003 |

| | | |
|---|---|---|
| * Viscosity measured at a shear rate of 21 1/s. | | |

## Claims

1. An aqueous based mouth wash oral care composition comprising:
i) a metal salt selected from citrate, lactate or chloride; and
ii) a zinc salt
iii) gellan gum
iv) xanthan gum
in which the composition at a temperature of 25°C has a yield stress (shear stress at a shear rate of 0.1 1/s) from 0.05 to 1.0 Pa and a viscosity from 0.05 to 0.25 Pa.s (measured at a shear rate of 21 1/s).

2. An oral care composition according to claim 1, in which the weight ratio of xanthan gum to gellan gum is from 2:1 to 6:1.

3. An oral care composition according to any preceding claim in which the level of xanthan gum is from 0.02 to 0.5 wt% of the total composition.

4. An oral care composition according to claim 1 in which the level of gellan gum is from 0.02 to 0.4 wt% of the total composition.

5. An oral care composition according to any preceding claim in which the metal salt (i) is citrate.

6. An oral care composition according to claim 5 in which the citrate salt is potassium citrate.

7. An oral care composition according to any preceding claim in which the zinc salt is zinc sulphate, zinc sulphate heptahydrate or zinc chloride.

8. An oral care composition according to any preceding claim in which the level of citrate salt is from 0.1 to 1 wt% of the total formulation.

9. An oral care composition according to any preceding claim in which the level of zinc salt is from 0.05 to 0.6 wt% of the total composition.

10. An oral care composition according to any preceding claim in which the composition at a temperature of 25°C has a yield stress (shear stress at a shear rate of 0.1 1/s at 25°C) from 0.1 to 0.5 Pa.

11. An oral care composition according to any preceding claim in which the composition at a temperature of 25°C has a viscosity from 0.07 to 0.15 Pa.s (measured at a shear rate of 21 1/s).

12. An oral care composition according to any preceding claim in which the pH of the composition at 20° C is from 4 .2 to 5.9.

13. An oral care composition according to any preceding claim in which the level of ethanol in the total composition is less than 0.1 wt%.

14. An oral care composition according to any preceding claim in which the level of anionic surfactant in the total composition is less than 0.35 wt%.

15. A method of reducing mouth odour by rinsing the mouth and teeth with a composition described in any preceding claims.

## Patentansprüche

1. Mundpflegezusammensetzung auf Wasserbasis zur Mundspülung, umfassend:
i) ein Metallsalz, ausgewählt unter Citrat, Lactat oder Chlorid, und
ii) ein Zinksalz,
iii) Gellangummi,
iv) Xanthangummi,
worin die Zusammensetzung bei einer Temperatur von 25°C eine Streckspannung (Scherspannung bei einer Scherrate von 0,1 1/s) von 0,05 bis 1,0 Pa und eine Viskosität von 0,05 bis 0,25 Pa.s (gemessen bei einer Scherrate von 21 1/s) aufweist.

2. Mundpflegezusammensetzung nach Anspruch 1, worin das Gewichtsverhältnis von Xanthangummi zu Gellangummi 2:1 bis 6:1 beträgt.

3. Mundpflegezusammensetzung nach irgendeinem vorhergehenden Anspruch, worin der Anteil des Xanthangummis 0,02 bis 0,5 Gew.-% der gesamten Zusammensetzung beträgt.

4. Mundpflegezusammensetzung nach Anspruch 1, worin der Anteil des Gellangummis 0,02 bis 0,4 Gew.-% der gesamten Zusammensetzung beträgt.

5. Mundpflegezusammensetzung nach irgendeinem vorhergehenden Anspruch, worin das Metallsalz (i) Citrat ist.

6. Mundpflegezusammensetzung nach Anspruch 5, worin das Citratsalz ein Kaliumcitrat ist.

7. Mundpflegezusammensetzung nach irgendeinem vorhergehenden Anspruch, worin das Zinksalz Zinksulfat, Zinksulfatheptahydrat oder Zinkchlorid ist.

8. Mundpflegezusammensetzung nach irgendeinem vorhergehenden Anspruch, worin der Anteil des Citratsalzes 0,1 bis 1 Gew.-% der gesamten Formulierung beträgt.

9. Mundpflegezusammensetzung nach irgendeinem vorhergehenden Anspruch, worin der Anteil des Zinksalzes 0,05 bis 0,6 Gew.-% der gesamten Zusammensetzung beträgt.

10. Mundpflegezusammensetzung nach irgendeinem vorhergehenden Anspruch, worin die Zusammensetzung bei einer Temperatur von 25°C eine Streckspannung (Scherspannung bei einer Scherrate von 0,1 1/s bei 25°C) von 0,1 bis 0,5 Pa aufweist.

11. Mundpflegezusammensetzung nach irgendeinem vorhergehenden Anspruch, worin die Zusammensetzung bei einer Temperatur von 25°C eine Viskosität von 0,07 bis 0,15 Pa.s (gemessen bei einer Scherrate von 21 1/s) aufweist.

12. Mundpflegezusammensetzung nach irgendeinem vorhergehenden Anspruch, worin der pH der Zusammensetzung bei 20°C 4,2 bis 5,9 beträgt.

13. Mundpflegezusammensetzung nach irgendeinem vorhergehenden Anspruch, worin der Anteil an Ethanol in der gesamten Zusammensetzung weniger als 0,1 Gew.-% beträgt.

14. Mundpflegezusammensetzung nach irgendeinem vorhergehenden Anspruch, worin der Anteil an anionischem Tensid in der gesamten Zusammensetzung weniger als 0,35 Gew.-% beträgt.

15. Verfahren zur Verminderung des Mundgeruchs durch Spülen des Mundes und der Zähne mit einer Zusammensetzung, wie in irgendeinem der vorhergehenden Ansprüche beschrieben.

## Revendications

1. Composition aqueuse pour soins buccaux-dentaires de type bain de bouche comprenant :
i) un sel métallique choisi parmi le citrate, le lactate ou le chlorure ; et
ii) un sel de zinc
iii) une gomme gellane
iv) une gomme xanthane
dans laquelle la composition à une température de 25 °C a une limite d'élasticité (contrainte de cisaillement à une vitesse de cisaillement de 0,1 1/s) de 0,05 à 1,0 Pa et une viscosité (mesurée à une vitesse de cisaillement de 21 1/s) de 0,05 à 0,25 Pa.s.

2. Composition pour soins buccaux-dentaires selon la revendication 1, dans laquelle le rapport en poids de la gomme xanthane à la gomme gellane est de 2,1 à 6,1.

3. Composition pour soins buccaux-dentaires selon l'une quelconque des revendications précédentes dans laquelle le niveau de gomme xanthane est de 0,02 à 0,5 % en poids de la composition totale.

4. Composition pour soins buccaux-dentaires selon la revendication 1 dans laquelle le niveau de gomme gellane est de 0,02 à 0,4 % en poids de la composition totale.

5. Composition pour soins buccaux-dentaires selon l'une quelconque des revendications précédentes dans laquelle le sel métallique (i) est un citrate.

6. Composition pour soins buccaux-dentaires selon la revendication 5 dans laquelle le sel de citrate est le citrate de potassium.

7. Composition pour soins buccaux-dentaires selon l'une quelconque des revendications précédentes dans laquelle le sel de zinc est un sulfate de zinc, un heptahydrate de sulfate de zinc ou un chlorure de zinc.

8. Composition pour soins buccaux-dentaires selon l'une quelconque des revendications précédentes dans laquelle le niveau de sel de citrate est de 0,1 à 1 % en poids de la formulation totale.

9. Composition pour soins buccaux-dentaires selon l'une quelconque des revendications précédentes dans laquelle le niveau de sel de zinc est de 0,05 à 0,6 % en poids de la composition totale.

10. Composition pour soins buccaux-dentaires selon l'une quelconque des revendications précédentes dans laquelle la composition à une température de 25 °C a une limite d'élasticité (contrainte de cisaillement à une vitesse de cisaillement de 0,1 1/s à 25 °C) de 0,1 à 0,5 Pa.

11. Composition pour soins buccaux-dentaires selon l'une quelconque des revendications précédentes dans laquelle la composition à une température de 25 °C a une viscosité (mesurée à une vitesse de cisaillement de 21 1/s) de 0,07 à 0,15 Pa.s.

12. Composition pour soins buccaux-dentaires selon l'une quelconque des revendications précédentes dans laquelle le pH de la composition à 20 °C est de 4,2 à 5,9.

13. Composition pour soins buccaux-dentaires selon l'une quelconque des revendications précédentes dans laquelle le niveau d'éthanol dans la composition totale est inférieur à 0,1 % en poids.

14. Composition pour soins buccaux-dentaires selon l'une quelconque des revendications précédentes dans laquelle le niveau de tensioactif anionique dans la composition totale est inférieur à 0,35 % en poids.

15. Méthode permettant de réduire une mauvaise haleine par rinçage de la cavité buccale et des dents avec une composition selon l'une quelconque des revendications précédentes.
